# EUROPÄISCHE PATENTSCHRIFT

(11) **EP 1 666 447 B1**
(45) Veröffentlichungstag und Bekanntmachung des Hinweises auf die Patenterteilung: **14.02.2007**
(21) Anmeldenummer: 05025658.5
(22) Anmeldetag: 24.11.2005
(51) Int. Cl.: C07C 51/367, C07C 67/31, C07C 231/02, C07C 235/00, C07C 59/01, C07C 69/675, C07C 62/00

(54) **Verfahren zur Herstellung von Alpha, Alpha-Dialkyl-Alpha-Hydroxymethyl-Carbonsäurederivaten**
Process for the preparation of alpha, alpha-dialkyl, alpha-hydroxymethyl-carboxylic acid derivatives
Procédé de préparation de dérives d'acides carboxyliques d'alpha, alpha-dialkyl, alpha-hydroxymethyl

(30) Priorität: 01.12.2004 DE 102004057995
(43) Veröffentlichungstag der Anmeldung: 07.06.2006
(73) Patentinhaber: Consortium für elektrochemische Industrie GmbH, 81379 München (DE)
(72) Erfinder: Böhm, Andreas, Dr., 81829 München (DE); Petersen, Hermann, Dr., 84489 Burghausen (DE); Stohrer, Jürgen, Dr., 82049 Pullach (DE)
(74) Vertreter: Fränkel, Robert

(56) Entgegenhaltungen:
- BAKER, S. R ET AL: "The Synthesis of [2.2.2]Bicyclooctane and [3.1.1]Bicycloheptane Based Amino Acids as Constrained Glutamate Analogues" TETRAHEDRON LETTERS, Bd. 40, 1999, Seite 781-784, XP002360456
- CLIVE, D. L. J. ET AL: "Synthesis of the racemic tetracyclic core of cP-225,917: use of a strain-assisted Cope rearrangement" TETRAHEDRON LETTERS, Bd. 43, , Seiten 4559-4563, XP002360457
- LEFEVRE, G. N. ET AL: "Intramolecular Steric Factors in the Thermolysis of 4-Alkylidene-1-pyrazolines" JOURNAL OF THE AMERICAN CHEMICAL SOCIETY, Bd. 108, 1986, Seiten 1019-1027, XP002360458

## Beschreibung

Die Erfindung betrifft ein neues Verfahren zur Hydroxymethylierung von nichtzyklischen α,α-Dialkyl-Carbonsäurederivaten mit Formaldehyd unter Verwendung von Amid-Basen bei Temperaturen von -40°C bis zum Siedepunkt des verwendeten Lösungsmittels oder Lösungsmittelgemisches.

Die Zielverbindungen sind als Intermediate zur Herstellung von Polymeren und pharmazeutischen Wirkstoffen geeignet und somit von großem technischen und wirtschaftlichen Interesse.

Aus dem Stand der Technik sind eine Reihe von Verfahren zur Hydroxymethylierung von α,α-Dialkyl-Carbonsäurederivaten bekannt. Allgemeines Prinzip der aus dem Stand der Technik bekannten Reaktionen ist der Einsatz von Formaldehyd als Hydroxymethylierungsreagenz in Gegenwart einer Base, überwiegend solchen aus der Klasse der Amidbasen, bei tiefen Temperaturen. Es ist insbesondere bekannt, dass α,α-Dialkyl-Carbonsäureester nach Deprotonierung mit Lithiumdiisopropylamid (LDA) mit Formaldehyd zu den entsprechenden α,α-Dialkyl-α-Hydroxymethyl-Carbonsäureestern bei tiefen Temperaturen umgesetzt werden können.

Derrick L. J. Clive et al. (Tetrahedron Letters 43 (2002), S. 4559-4563) und Gerard N. LeFevre et al. (Journal of the American Chemical Society 108 (1986), S. 1019-1027) beschreiben jeweils ein Verfahren zur Hydroxymethylierung eines Norbornenderivates mit Paraformaldehyd. Sowohl für die Erzeugung des Enolats des als Ausgangsverbindung eingesetzten Esters (Ester-Enolat) mit LDA als auch bei der weiteren Umsetzung des so erzeugten Enolats mit Paraformaldehyd müssen, um eine signifikante Umsetzung zu erreichen, sehr tiefe Temperaturen von -78°C angewandt werden. Die Produktausbeuten liegen dennoch bei lediglich 43 % bzw. 61 %.

Die Umsetzung strukturell eng verwandter Cylohexansäure- und Cyclopentansäureester mit Lithiumdiisopropylamid und gasförmigem Formaldehyd ist von Michiharu Kato et al. (Chemistry Letters (1985), S. 1785-1788), S. Richard Baker et al. (Tetrahedron Letters 40 (1999), S. 781-784) und B. R. Neustadt et al. (J. Med. Chem. 37 (1994), S. 2461-2467) beschrieben worden. Sowohl für die Erzeugung des Ester-Enolats mit LDA als auch für die weitere Umsetzung mit Formaldehyd werden auch nach diesem Verfahren wiederum sehr tiefe Temperaturen von -78°C angewandt.

Die Umsetzung eines α,α-Dialkyl-Carbonsäureesters bei höheren Temperaturen wurde einzig durch die Verwendung einer sehr speziellen und teuren Base erreicht, da anderenfalls keinerlei Umsetzung bei Nicht-Tieftemperaturbedingungen beobachtet werden konnte. George R. Pettit et al. (Synthetic Communications 11(3), 1981, S. 167-177) beschreibt die Deprotonierung eines Cyclohexancarbonsäureesters mit Trityllithium und anschließende Hydroxymethylierung des gebildeten Enolates mit Paraformaldehyd bei Eisbad-Temperaturen. Die Autoren vermuteten, dass die Bildung des Carbanions in diesem Fall besonders schwierig ist und deshalb eine spezielle Base zur Deprotonierung, wie Trityllithium, eingesetzt werden muss.

Die Hydroxymethylierung einer freien α,α-Dialkyl-Carbonsäure durch Generierung des Enolates mit einem Überschuss an LDA und Umsetzung mit gasförmigem Formaldehyd ist am Beispiel eines Cyclohexancarbonsäurederivates von F. E. Ziegler et al. (Organic Letters 2 (2000), S. 3619-3622) beschrieben worden. Wiederum müssen allerdings auch in diesem Verfahren sehr tiefe Temperaturen von -78°C angewandt werden.

In Kenntnis des Stands der Technik kann der Fachmann somit den Rückschluss ziehen, dass für die erfolgreiche Hydroxymethylierung der darin beschriebenen α,α-Dialkyl-Carbonsäurederivate mit Formaldehyd unter Einsatz von Amid-Basen Temperaturen von -78°C zwingend eingehalten werden müssen. Werden höhere Reaktionstemperaturen angestrebt, so ist eine Umsetzung lediglich bei Verwendung spezieller und teurer Basen, wie beispielsweise Trityllithium, zu erreichen.

Für eine großtechnische oder industrielle Umsetzung der Hydroxymethylierung von α,α-Dialkyl-Carbonsäurederivaten ist jedoch sowohl die Notwendigkeit der Anwendung von Tieftemperaturbedingungen und/oder der Einsatz spezieller und kostspieliger Reagenzien mit großen technischen und wirtschaftlichen Nachteilen behaftet.

Aus dem Stand der Technik sind weiter keine analogen Hydroxymethylierungsreaktionen von α,α-Dialkyl-Carbonsäurederivaten bekannt, in denen das in α-Stellung zur Carboxyl-Gruppe befindliche Kohlenstoffatom nicht Teil eines zyklischen Kohlenwasserstoffsystems ist, da sich die im Stand der Technik beschriebenen Substrate ausschließlich von Cyclopentan-, Cyclohexan- oder Norbornen-Carbonsäurederivaten ableiten. Die Hydroxymethylierung von α,α-Dialkyl-Carbonsäurederivaten, die dieses Strukturmerkmal nicht aufweisen, ist im Stand der Technik bislang nicht beschrieben worden. Insbesondere ist die Hydroxymethylierung offenkettiger Substrate, wie beispielsweise 2-Ethylhexansäure und deren Derivate, mit Formaldehyd im Stand der Technik bislang noch nicht beschrieben worden.

Der Stand der Technik stellte dem Fachmann keinen Hinweis zur Verfügung, ob die Hydroxymethylierung von α,α-Dialkyl-Carbonsäurederivaten, in denen das in α-Stellung zur Carboxyl-Gruppe befindliche Kohlenstoffatom nicht Teil eines zyklischen Kohlenwasserstoffsystems ist, überhaupt möglich ist und wie im Einzelnen mit solchen Substraten zu verfahren ist. Vielmehr gab der Stand der Technik dem Fachmann Veranlassung, davon auszugehen, dass solche Hydroxymethylierungen ausschließlich mit Carbonsäurederivaten, in denen das in α-Stellung zur Carboxyl-Gruppe befindliche Kohlenstoffatom Teil eines zyklischen Kohlenwasserstoffsystems ist, unter Tieftemperaturbedingungen oder unter Verwendung von speziellen Reagenzien möglich ist.

Es bestand daher die Aufgabe ein weiteres Verfahren zur Hydroxymethylierung von α,α-Dialkyl-Carbonsäurederivaten bereitzustellen, das die aus den Verfahren des Stands der Technik bekannten Probleme vermeidet.

Es bestand insbesondere die Aufgabe, ein Verfahren zur Herstellung von α,α-Dialkyl-α-Hydroxymethyl-Carbonsäurederivaten ausgehend von α,α-Dialkyl-Carbonsäurederivaten bereitzustellen, in denen das in α-Stellung zur Carboxyl-Gruppe befindliche Kohlenstoffatom nicht Teil eines zyklischen Kohlenwasserstoffsystems (Zyklus, Ringsystems) ist.

Die Aufgabe wurde gelöst durch ein Verfahren zur Herstellung von α,α-Dialkyl-α-Hydroxymethyl-Carbonsäurederivaten ausgehend von α,α-Dialkyl-Carbonsäurederivaten, in denen das in α-Stellung befindliche und der Hydroxymethylierung zu unterwerfende Kohlenstoffatom nicht Teil eines Zyklus ist, durch Umsetzung mit Formaldehyd in Gegenwart einer Amid-Base bei Reaktionstemperaturen von -40°C bis zum Siedepunkt des verwendeten Lösungsmittels oder Lösungsmittelgemisches.

Es wurde überraschend gefunden, dass unter diesen Reaktionsbedingungen solche Substrate mit hohen Ausbeuten und Reinheiten zu den Zielverbindungen umgesetzt werden können. Das erfindungsgemäße Verfahren zeichnet sich insbesondere dadurch aus, dass mit großtechnisch gängigen Basen, insbesondere Amid-Basen, bei vergleichsweise unkomplizierten Reationstemperaturen operiert werden kann. Auf den Einsatz spezieller und teurer Basen wie Trityllithium, die Notwendigkeit der Reaktionsdurchführung bei sehr tiefen Temperaturen von -78°C kann verzichtet werden. Ferner eröffnet das erfindungsgemäße Verfahren erstmals die Möglichkeit, auch Carbonsäurederivate, die sich nicht von Cyclopentan-, Cyclohexan- oder Norbornen-Verbindungen ableiten einer Hydroxymethylierung mit Formaldehyd zu unterwerfen.

Gegenstand der Erfindung ist ein Verfahren zur Herstellung von α,α-Dialkyl-α-Hydroxymethyl-Carbonsäurederivaten oder deren Salzen der allgemeinen Formel (II) 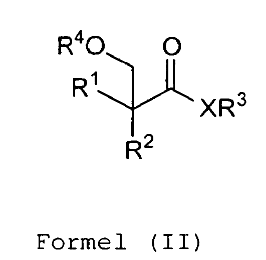 wobei
R¹ und R² unabhängig voneinander ausgewählt werden aus der Gruppe enthaltend lineare oder verzweigte Alkyl-Reste und Aralkyl-Reste,
R³ ausgewählt wird aus der Gruppe enthaltend Wasserstoff, gegebenenfalls substituierte lineare oder verzweigte Alkyl-, Aryl-, Aralkyl-Reste, Silyl-Reste, Alkalimetall, Erdalkalimetall und Ammonium,
R⁴ für Wasserstoff, eine Hydroxyl-Schutzgruppe, Alkalimetall oder Erdalkalimetall steht,
X ausgewählt wird aus der Gruppe enthaltend Sauerstoff, Schwefel und NR⁵, wobei
R⁵ wiederum ausgewählt wird aus der Gruppe enthaltend Wasserstoff, lineare oder verzweigte Alkyl-, Aryl-, Aralkyl-Reste und Silyl-Reste,
durch Umsetzung von α,α-Dialkyl-Carbonsäurederivaten oder deren Salze der allgemeinen Formel (I) 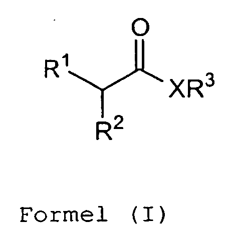 mit Formaldehyd in Gegenwart einer Amid-Base in einem oder mehreren Lösungsmitteln, dadurch gekennzeichnet, dass die Umsetzung in einem Temperaturbereich von -40°C bis zum Siedepunkt des verwendeten Lösungsmittels oder Lösungsmittelgemisches erfolgt.

Das erfindungsgemäße Verfahren bietet erstmals die Möglichkeit zur Hydroxymethylierung von Substraten der allgemeinen Formel (I) und folglich Zugang zu Produkten der allgemeinen Formel (II), in denen das Kohlenstoffatom, an dem die Hydroxymethylen-Gruppierung eingeführt wird, nicht Teil eines Ringsystems ist, insbesondere die Reste R¹ und R² in den allgemeinen Formeln (I) und (II) kein geschlossenes Ringsystem, insbesondere keinen Kohlenwasserstoff-Ring bilden und die Reste R¹ und R² somit nicht miteinander verknüpft sind.

Die erfindungsgemäße Hydroxymethylierung von α,α-Dialkyl-Carbonsäurederivaten oder deren Salzen der allgemeinen Formel (I) wird derart durchgeführt, dass im erfindungsgemäßen Temperaturbereich zunächst das Edukt der allgemeinen Formel (I) in einem geeigneten Lösungsmittel oder Lösungsmittelgemisch mit einer Amid-Base in α-Stellung zur Carbonsäurefunktion deprotoniert und anschließend das so erhaltene reaktive Intermediat (Enolat) mit Formaldehyd zur Reaktion gebracht wird.

Alternativ kann auch das α,α-Dialkyl-Carbonsäurederivat oder deren Salze der allgemeinen Formel (I) zusammen mit dem Formaldehyd vorgelegt und die Umsetzung durch anschließende Zugabe der Amid-Base erfolgen.

Möglich ist auch die Vorlage der Amid-Base und des Formaldehyds und folgende Zugabe des α,α-Dialkyl-Carbonsäurederivats.

Bevorzugte Reste für R¹ und R² werden unabhängig voneinander ausgewählt aus der Gruppe enthaltend lineare oder verzweigte Alkyl- und Aralkyl-Reste, besonders bevorzugt und unabhängig voneinander ausgewählt aus der Gruppe enthaltend Methyl, Ethyl, n-Propyl, iso-Propyl, n-Butyl, iso-Butyl, tert-Butyl, n-Pentyl, n-Hexyl und Benzyl.

Im Falle, dass R³ für einen substituierten linearen oder verzweigten Alkyl-, Aryl-, Aralkyl-Rest steht, können solche Substituenten bevorzugt aus funktionellen Gruppen ausgewählt aus der Gruppe enthaltend Halogen, Cyano und Alkoxy, insbesondere aus der Gruppe der Halogene, ausgewählt werden.

Im Falle, dass R³ oder R⁵ für Silyl-Reste stehen, handelt es sich bevorzugt um Dialkylsilyl- oder Trialkylsilyl-Reste, wobei die darin enthaltenen Alkyl-Reste linear oder verzweigt sein können.

Bevorzugte Reste für R³ werden ausgewählt aus der Gruppe enthaltend Methyl, Ethyl, n-Propyl, iso-Propyl, n-Butyl, iso-Butyl, tert-Butyl, Benzyl und Trimethylsilyl.

Bevorzugte Reste für R⁵ werden ausgewählt aus der Gruppe enthaltend Methyl, Ethyl, n-Propyl, iso-Propyl, n-Butyl, iso-Butyl, tert-Butyl, Benzyl und Trimethylsilyl.

Bevorzugte Salze der α,α-Dialkyl-Carbonsäurederivate der allgemeinen Formel (I) sind solche, in denen R³ für Lithium, Natrium, Kalium, Magnesium, Calcium oder Tetraalkylammonium steht.

Unmittelbare Verfahrensprodukte sind die sich von den eingesetzten α,α-Dialkyl-Carbonsäurederivaten bzw. deren Salzen der allgemeinen Formel (I) ableitenden, anionischen, in α-Position Oxymethylen-funktionalisierten Zielverbindungen (Alkoholate) der allgemeinen Formel (II), wobei R⁴ zunächst für das aus der eingesetzten Amid-Base stammende Kation, insbesondere für Lithium oder Natrium steht, und durch wässrige Aufarbeitung eine Hydroxyfunktion erhalten wird, in der R⁴ für Wasserstoff steht.

Auch ist es möglich, die Hydroxymethyl-Verbindungen weiter mit Reagenzien zur Einführung von Hydroxyl-Schutzgruppen oder Abgangsgruppen umzusetzen, insbesondere z. B. mit Acyl-, Silyl-, Benzyl-, Mesyl- oder Tosyl-Gruppen weiter zu funktionalisieren bzw. zu derivatisieren. R⁴ kann demnach ganz allgemein für jede dem Fachmann bekannte Alkohol-Schutzgruppe (Hydroxyl-Schutzgruppe) stehen. Diese Funktionalisierung bzw. Derivatisierung kann auch im Zuge der Aufarbeitung der unmittelbaren Verfahrensprodukte durch den Fachmann bekannte und in der organischen Synthese übliche Verfahren ohne vorherige Überführung in oder Isolierung der Hydroxymethyl-Verbindung erfolgen.

Bevorzugte Hydroxyl-Schutzgruppen für R⁴ sind solche, die ausgewählt werden aus der Gruppe enthaltend, gegebenenfalls substituierte lineare oder verzweigte Alkyl-, Acyl-, Aryl- oder Aralkyl-Reste und Silyl-Reste, insbesondere Dialkylsilyl-Reste und Trialkylsilyl-Reste.

Im Falle, dass R⁴ für einen substituierten linearen oder verzweigten Alkyl-, Aryl-, Aralkyl-Rest steht, können solche Substituenten bevorzugt aus funktionellen Gruppen ausgewählt aus der Gruppe enthaltend Halogen, Cyano und Alkoxy, insbesondere aus der Gruppe der Halogene, ausgewählt werden.

Im Falle, dass R⁴ für einen Dialkylsilyl- oder Trialkylsilyl-Rest steht, können die darin enthaltenen Alkylreste linear oder verzweigt sein. Bevorzugte Silyl-Reste für R⁴ sind Trialkylsilyl-Reste mit 3 bis 12 Kohlenstoffatomen, insbesondere Trimethylsilyl.

Konkrete, besonders bevorzugte Ausführungsformen für Hydroxyl-Schutzgruppen für R⁴ sind Acetyl-, Trimethylsilyl-, Benzyl-, Mesyl- oder Tosyl-Reste.

In einer besonderen Ausführungsform des erfindungsgemäßen Verfahrens werden die bei der Reaktion gebildeten unmittelbaren Verfahrensprodukte (Primärprodukte) vor der weiteren, in der Regel wässrig/organischen Aufarbeitung, derivatisiert. So können die in der Reaktionsmischung zunächst vorliegenden Alkoholate und/oder gegebenfalls auch Carboxylate der allgemeinen Formel (II) direkt im Anschluss an die Reaktion durch Zugabe geeigneter Reagenzien derivatisiert bzw. funktionalisiert werden.

Zur Derivatisierung bzw. Funktionalisierung sind insbesondere alle im Stand der Technik bekannten Schutzgruppen für Alkohole und Carbonsäuren geeignet, insbesondere die aus Protective Groups in Organic Synthesis, 3rd Edition, editors T. W. Greene und P. G. M. Wuts, John Wiley & Sons, Inc. 1999 bekannten Gruppen.

Bevorzugt werden die in der Reaktionsmischung zunächst vorliegenden Alkoholate und/oder gegebenenfalls auch Carboxylate der Formel (II) direkt im Anschluss an die Reaktion durch Zugabe von Chlorsilanen, Mesylchlorid, Tosylchlorid, Benzylchlorid oder Benzylbromid derivatisiert. Besonders bevorzugt werden hierbei Trimethylchlorsilan und Dichlordimethylsilan verwendet.

Die Derivatisierung kann selbstverständlich auch mit den entsprechend protonierten Alkoholen und Carbonsäuren durchgeführt werden, in denen R³ und/oder R⁴ für Wasserstoff stehen.

Bevorzugte Edukte der allgemeinen Formel (I) sind α,α-Dialkyl-Carbonsäureester und die freien α,α-Dialkyl-Carbonsäuren oder deren Salze, wobei X für Sauerstoff (O) steht und R³ ausgewählt wird aus der Gruppe enthaltend Wasserstoff, gegebenenfalls substituierte lineare oder verzweigte Alkyl-, Aryl-, Aralkyl-Reste und Silyl-Reste, insbesondere aus der Gruppe enthaltend Wasserstoff, Methyl, Ethyl, n-Propyl, iso-Propyl, n-Butyl, iso-Butyl, tert-Butyl, Benzyl und Trimethylsilyl.

Werden die vorgenannten α,α-Dialkyl-Carbonsäureester oder die freien α,α-Dialkyl-Carbonsäuren als Edukte eingesetzt und steht R³ für einen Silyl-Rest, so ist dieser Silyl-Rest bevorzugt ein Dialkylsilyl- oder Trialkylsilyl-Rest, wobei die Alkyl-Reste hierin linear oder verzweigt sein können.

Neben den freien α,α-Dialkyl-Carbonsäuren sind auch deren Salze, in denen X für Sauerstoff (O) steht und R³ ausgewählt wird aus der Gruppe enthaltend Alkalimetalle, Erdalkalimetalle und Ammonium, insbesondere aus der Gruppe enthaltend Lithium, Natrium, Kalium, Magnesium, Calcium und Tetraalkylammonium, bevorzugte Edukte der allgemeinen Formel (I).

Besonders bevorzugte Edukte der allgemeinen Formel (I) sind α,α-Dialkyl-Carbonsäureester, wobei X für Sauerstoff (O) steht und R³ ausgewählt wird aus der Gruppe enthaltend gegebenenfalls substituierte lineare oder verzweigte Alkyl-, Aryl-, Aralkyl-Reste und Silyl-Reste. Besonders bevorzugt wird R³ hierbei aus der Gruppe enthaltend Methyl, Ethyl, n-Propyl, iso-Propyl, n-Butyl, iso-Butyl, tert-Butyl, Benzyl und Trimethylsilyl ausgewählt.

Unter den vorgenannten besonders bevorzugten Edukten sind wiederum solche besonders bevorzugt, in denen die Reste R¹ und R² aus den oben genannten besonders bevorzugten Ausführungsformen dieser Reste ausgewählt werden.

Die Salze der α,α-Dialkyl-Carbonsäuren können hierbei entweder direkt in der Reaktion eingesetzt oder auch in einfacher Weise in situ aus den korrespondierenden Carbonsäuren durch Zugabe einer geeigneten Base hergestellt werden. Als Base eignen sich hierzu beispielsweise Metallhydroxide, -amide, -carbonate, -hydrogencarbonate, und -hydride. Besonders bevorzugt werden hierzu Natriumhydrid, Lithiumhydrid, Natriumhydroxid und Lithiumhydroxid als Basen eingesetzt. Das gegebenenfalls hierbei gebildete Reaktionswasser kann vor der Erzeugung des Enolates beispielsweise durch azeotrope Destillation unter Verwendung eines geeigneten Lösungsmittels, wie beispielsweise Toluol, Pentan, MTBE und Methylenchlorid aus der Reaktionsmischung entfernt werden.

In einer besonders einfachen Ausführungsform der Hydroxymethylierung von α,α-Dialkyl-Carbonsäuren wird sowohl zur Deprotonierung der Carbonsäurefunktion -COOH als auch zur Erzeugung des Enolates eine Amid-Base verwendet. Dementsprechend sind demnach mindestens 2 Äquivalente der eingesetzten Amid-Base zur zweifachen Deprotonierung notwendig.

Als Amid-Basen sind prinzipiell alle dem Fachmann aus dem Stand der Technik bekannten und in der organischen Synthese typischerweise eingesetzten Metallamide geeignet. Bevorzugt eignen sich Metallamide der allgemeinen Formel (III)

M(NR⁶R⁷)ₙ Formel (III)

wobei
M ausgewählt wird aus der Gruppe enthaltend Lithium, Natrium, Kalium, Magnesium und Calcium und
R⁶ und R⁷ unabhängig voneinander ausgewählt werden aus der Gruppe enthaltend Wasserstoff, lineare oder verzweigte Alkyl-, Aryl-, Aralkyl-Reste und Silyl-Reste und
n in Abhängigkeit der Wertigkeit von M für 1 oder 2 steht.

Falls R⁶ und R⁷ unabhängig voneinander für einen oder mehrere Silyl-Reste stehen, handelt es sich bevorzugt um Trialkylsilyl-Reste, wobei die darin enthaltenen Alkyl-Reste linear oder verzweigt sein können.

Bevorzugt werden R⁶ und R⁷ unabhängig voneinander ausgewählt aus der Gruppe enthaltend Methyl, Ethyl, n-Propyl, iso-Propyl, n-Butyl, iso-Butyl, tert-Butyl, Benzyl und Trimethylsilyl.

Besonders bevorzugt wird Natriumhexamethyldisilazid, Lithiumhexamethyldisilazid, Lithiumdiisopropylamid und Lithiumtetramethylpiperidin als Amid-Basen eingesetzt.

Ganz besonders bevorzugt wird Lithiumdiisopropylamid als Amid-Base verwendet.

Bevorzugt werden 0.5 bis 10 und besonders bevorzugt werden 1-3 Äquivalente der Amid-Base bezogen auf das α,α-Dialkyl-Carbonsäurederivat eingesetzt. Zu beachten ist, dass bei Einsatz der freien α,α-Dialkyl-Carbonsäuren ein zusätzliches Äquivalent Base zunächst für die Deprotonierung der COOH-Gruppe verbraucht wird. Alternativ kann die COOH-Gruppe zunächst auch mit alternativen Basen deprotoniert werden (siehe oben).

Formaldehyd als das Reagenz zur Hydroxymethylierung kann prinzipiell in alle denkbaren und in der organischen Synthese üblichen Formen eingesetzt werden, insbesondere als freier gasförmiger Formaldehyd, Paraformaldehyd oder auch als Trioxan.

Bei Verwendung von freiem gasförmigen Formaldehyd wird dieser vorzugsweise durch Depolymerisation von beispielsweise Paraformaldehyd bei hohen Temperaturen von T > 150°C erzeugt, wie es beispielsweise aus B. R. Neustadt et al., J. Med. Chem. 37 (1994), S. 2461-2467 bekannt ist, was jedoch einen bedeutenden zusätzlichen Verfahrensaufwand darstellt. Auch ist das Handling von toxischem Formaldehyd-Gas aus Sicherheitsgründen problematisch. Bevorzugt wird deshalb Paraformaldehyd oder Trioxan und ganz besonders bevorzugt Paraformaldehyd eingesetzt, da bei Einsatz von Trioxan häufig deutlich schlechtere Ausbeuten der Produkte erhalten werden.

Von Paraformaldehyd können verschiedene Qualitäten und unterschiedliche mittlere Kettenlängen des technisch und kostengünstig verfügbaren Paraformaldehyds eingesetzt werden.

Bevorzugt werden 0.5 - 10, besonders bevorzugt werden 1 - 5 und ganz besonders bevorzugt werden 1 - 3 Äquivalente Formaldehyd bezogen auf das α,α-Dialkyl-Carbonsäurederivat eingesetzt.

Für das erfindungsgemäße Verfahren werden bevorzugt Lösungsmittel, ausgewählt aus der Gruppe enthaltend Tetrahydrofuran, Diethylether, MTBE, Dibutylether, Toluol, Methylenchlorid, Pentan, Hexan, Heptan und Petrolether sowie deren Mischungen eingesetzt. Besonders bevorzugt werden Tetrahydrofuran, MTBE, Dibutylether, Pentan, Hexan oder Heptan sowie deren Mischungen und ganz besonders bevorzugt Tetrahydrofuran sowie Mischungen mit Pentan, Hexan und/oder Heptan eingesetzt.

Die erfindungsgemäße Hydroxymethylierung von α,α-Dialkyl-Carbonsäurederivaten der allgemeinen Formel (I) und deren Salzen wird im allgemeinen bei Temperaturen von T = -40°C bis zum Siedepunkt des verwendeten Lösungsmittels oder Lösungsmittelgemisches durchgeführt. Diese Temperaturen sind ohne größeren technischen und wirtschaftlichen Aufwand zu realisieren. Tieftemperaturbedingungen, wie sie im Stand der Technik für die Umsetzung von α,α-Dialkyl-Carbonsäurederivaten mit Formaldehyd unter Verwendung von Amid-Basen beschrieben wurden (T = -78°C), bedeuten im Vergleich hierzu einen deutlich höheren verfahrenstechnischen und somit kostenintensiveren Aufwand.

Besonders bevorzugt wird die Hydroxymethylierung von α,α-Dialkyl-Carbonsäurederivaten der allgemeinen Formel (I) und deren Salzen bei Reaktionstemperaturen von -30°C bis +60°C durchgeführt. Dies bedeutet einen erheblichen Vorteil für die Wirtschaftlichkeit des erfindungsgemäßen Verfahrens. Besonders überraschend ist hierbei, dass bei der Durchführung im erfindungsgemäßen Temperaturbereich sehr gute Ausbeuten (bis zu >90%) und hohe Reinheiten der Produkte erhalten werden können.

Als Produkte der Hydroxymethylierung von α,α-Dialkyl-Carbonsäurederivaten und deren Salzen der allgemeinen Formel (I) werden α,α-Dialkyl-α-Hydroxymethyl-Carbonsäurederivate oder deren Salze der allgemeinen Formel (II) erhalten 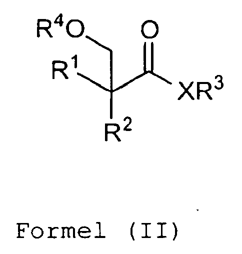 wobei die Reste R¹, R², R³, R⁴, R⁵ und X die oben genannte Bedeutung haben, insbesondere aus den oben genannten bevorzugten Ausführungsformen bestehen.

Insbesondere lassen sich durch das Verfahren aus 2-Ethylhexansäure 2-Ethyl-2-hydroxymethyl-hexansäure und aus 2-Ethylhexansäure-methylester 2-Ethyl-2-hydroxymethyl-hexansäure-methylester erhalten (siehe Beispiele).

Die folgenden Beispiele dienen der detaillierten Erläuterung der Erfindung und sind in keiner Weise als Einschränkung zu verstehen.

### Beispiele

### Beispiel 1 (2-Ethyl-2-Hydroxymethyl-Hexansäuremethylester)

Zu einer Lösung von 118 ml (0.319 Mol) n-Butyl-Lithium (2.7 M in Heptan) in 118 ml THF werden bei -20°C 47 ml (0.334 Mol) Diisopropylamin zugetropft und 30 min bei - 20°C gerührt. Innerhalb von ca. 15 min werden bei -20°C 48 g (0.304 Mol) 2-Ethylhexansäuremethylester zugetropft, 30 min bei -20°C nachgerührt und anschließend mit 27 g (0.90 Mol) Paraformaldehyd versetzt. Nach 30 min bei -20°C wird 2h bei 0°C nachgerührt. Nach dem Entfernen des Lösungsmittels bei 20°C und 20 mbar (V (Destillat) = ca. 250 ml) werden zu dem öligen Rückstand 100 ml MTBE und 200 ml gesättigte wässrige Lösung von Ammoniumchlorid gegeben und 2h bei RT gerührt. Die organische Phase wird abgetrennt, die wässrige Phase 1x mit 100 ml MTBE nachextrahiert und die vereinigten organischen Phasen am Rotationsverdampfer eingeengt. Das Rohprodukt (97 % Umsatz bezogen auf 2-Ethylhexansäuremethylester) kann durch Destillation aufgereinigt werden, wobei 2-Ethyl-2-Hydroxymethyl-Hexansäuremethylester (Sdp.: 100°C, 6 mbar) in 91 % Ausbeute mit einer Reinheit von > 99 % erhalten wird.

### Beispiel 2 (2-Ethyl-2-Hydroxymethyl-Hexansäuremethylester)

Zu einer Lösung von 12.9 ml (0.035 Mol) n-Butyl-Lithium (2.7 M in Heptan) in 17 ml THF werden bei 0°C 5.2 ml (0.037 Mol) Diisopropylamin zugetropft und 15 min bei 0°C gerührt. Innerhalb von ca. 10 min werden bei 0°C 5 g (0.032 Mol) 2-Ethylhexansäuremethylester zugetropft, 30 min bei 0°C nachgerührt und anschließend mit 2.9 g (0.097 Mol) Paraformaldehyd versetzt. Nach 2h Rühren bei 0°C wird eine gesättigte wässrige Lösung von Ammoniumchlorid (20 ml) zugegeben und 2h bei RT gerührt. Die organische Phase wird abgetrennt, die wässrige Phase einmal mit 20 ml MTBE nachextrahiert und die vereinigten organischen Phasen am Rotationsverdampfer eingeengt. Die Ausbeute an reinem Produkt liegt bei 83 % (GC).

### Beispiel 3 (2-Ethyl-2-Hydroxymethyl-Hexansäuremethylester)

Zu einer Lösung von 12.9 ml (0.035 Mol) n-Butyl-Lithium (2.7 M in Heptan) in 17 ml THF werden bei 20°C 5.2 ml (0.037 Mol) Diisopropylamin zugetropft und 15 min bei 20°C gerührt. Innerhalb von ca. 10 min werden bei 20°C 5g (0.032 Mol) 2-Ethylhexansäuremethylester zugetropft, 30 min bei 20°C nachgerührt und anschließend mit 2.9 g (0.097 Mol) Paraformaldehyd versetzt. Nach 2h Rühren bei 20°C wird eine gesättigte wässrige Lösung von Ammoniumchlorid (20 ml) zugegeben und 2h bei RT gerührt. Die organische Phase wird abgetrennt, die wässrige Phase 1x mit 20 ml MTBE nachextrahiert und die vereinigten organischen Phasen am Rotationsverdampfer eingeengt. Die Ausbeute an reinem Produkt liegt bei 77 % (GC).

### Beispiel 4 (2-Ethyl-2-Hydroxymethyl-Hexansäuremethylester)

Zu einer Lösung von 24.4 ml (0.065 Mol) n-Butyl-Lithium (2.7 M in Heptan) in 25 ml MTBE werden bei -20°C 9.8 ml (0.070 Mol) Diisopropylamin zugetropft und 30 min bei -20°C gerührt. Innerhalb von ca. 10 min werden bei -20°C 10 g (0.063 Mol) 2-Ethylhexansäuremethylester zugetropft, 30 min bei -20°C nachgerührt und anschließend mit 5.7 g (0.19 Mol) Paraformaldehyd versetzt. Nach 30 min bei -20°C wird 2h bei 0°C nachgerührt. Nach dem Entfernen des Lösungsmittels bei 20°C und 20 mbar werden zu dem öligen Rückstand (ca. 20g) 20 ml MTBE und 40 ml gesättigte wässrige Lösung von Ammoniumchlorid gegeben und 2h bei RT gerührt. Die organische Phase wird abgetrennt, die wässrige Phase 1x mit 20 ml MTBE nachextrahiert und die vereinigten organischen Phasen am Rotationsverdampfer eingeengt. Die Ausbeute an reinem Produkt liegt bei 86 % (GC).

### Beispiel 5 (2-Ethyl-2-Hydroxymethyl-Hexansäuremethylester)

Zu einer Lösung von 118 ml (0.319 Mol) n-Butyl-Lithium (2.7 M in Heptan) in 118 ml THF werden bei -20°C 47 ml (0.334 Mol) Diisopropylamin zugetropft und 30 min bei -20°C gerührt. Innerhalb von ca. 15 min werden bei -20°C 48 g (0.304 Mol) 2-Ethylhexansäuremethylester zugetropft, 30 min bei -20°C nachgerührt und anschließend mit 18 g (0.60 Mol) Paraformaldehyd versetzt. Nach 30 min bei -20°C wird 2h bei 0°C nachgerührt. Nach Zugabe von 100 ml MTBE und 100 ml gesättigter wässriger Lösung von Ammoniumchlorid wird 2h bei RT gerührt. Die organische Phase wird abgetrennt, die wässrige Phase 1x mit 100 ml MTBE nachextrahiert und die vereinigten organischen Phasen am Rotationsverdampfer eingeengt. Das Rohprodukt (97% Umsatz bezogen auf 2-Ethylhexansäuremethylester) kann durch Destillation aufgereinigt werden, wobei 2-Ethyl-2-Hydroxymethyl-Hexansäuremethylester (Sdp.: 100°C, 6 mbar) in 91 % Ausbeute mit einer Reinheit von > 99% erhalten wird.

### Beispiel 6 (2-Ethyl-2-Hydroxymethyl-Hexansäure)

Zu einer Lösung von 10.9 ml Diisopropylamin (77.5 mmol) in 60 ml THF werden bei 0°C 27 ml (72.9 mmol) n-Butyl-Lithium (2.7 M in Heptan) zugetropft und 30 min bei 0°C gerührt. Innerhalb von 10 min werden bei 0°C 5g (34.7 mmol) 2-Ethylhexansäure zugetropft und die Reaktionsmischung 1.5h auf 50°C erwärmt. Die klare Lösung wird auf -20°C abgekühlt und mit 3.1g (103 mmol) Paraformaldehyd versetzt. Nach 10 min bei -20°C wird 2h bei RT nachgerührt. Es wird mit gesättigter wässriger Lösung von Ammoniumchlorid versetzt und mit 6N HCI auf pH = 1-2 eingestellt. Die organische Phase wird abgetrennt, die wässrige Phase einmal mit MTBE nachextrahiert und die vereinigten organischen Phasen am Rotationsverdampfer eingeengt. Das Rohprodukt (85 % Ausbeute (GC)) kann durch Destillation weiter aufgereinigt werden (Sdp.: 121°C, 0.2 mbar). Alternativ ist es auch möglich, das Natriumsalz des Produktes aus wässriger Lösung in reiner Form zu kristallisieren. Das reine Produkt der freien 2-Ethyl-2-Hydroxymethyl-Hexansäure, welches üblicherweise als viskoses Öl erhalten wird, kristallisiert langsam bei RT (Schmelzpunkt: 45 - 46°C). Alternativ kann das Produkt auch aus einem geeigneten Lösungsmittel, wie beispielsweise Pentan, Hexan, Heptan oder Petrolether kristallisiert werden.

### Beispiel 7 (2-Ethyl-2-Hydroxymethyl-Hexansäure)

Zu einer Lösung von 10.9 ml Diisopropylamin (77.5 mmol) in 60 ml THF werden bei 0°C 27 ml (72.9 mmol) n-Butyl-Lithium (2.7 M in Heptan) zugetropft und 30 min bei 0°C gerührt. Innerhalb von 10 min werden bei 0°C 5g (34.7 mmol) 2-Ethylhexansäure zugetropft und die Reaktionsmischung 1.5h auf 50°C erwärmt. Die klare Lösung wird auf 0°C abgekühlt und mit 3.1 g (103 mmol) Paraformaldehyd versetzt. Nach 10 min bei 0°C wird 18h bei RT nachgerührt. Es wird mit gesättigter wässriger Lösung von Ammoniumchlorid versetzt und mit 6N HCI auf pH = 1-2 eingestellt. Die organische Phase wird abgetrennt, die wässrige Phase einaml mit MTBE nachextrahiert und die vereinigten organischen Phasen am Rotationsverdampfer eingeengt. Die Ausbeute an reinem Produkt liegt bei 89 % (GC).

### Beispiel 8 (2-Ethyl-2-Hydroxymethyl-Hexansäure)

Zu einer Lösung von 46 ml Diisopropylamin (0.327 Mol) in 320 ml THF werden bei 0°C 113 ml (0.305 Mol) n-Butyl-Lithium (2.7 M in Heptan) zugetropft und 30 min bei 0°C gerührt (Lösung 1). Zu einer Suspension von 7 g NaH 95 % (0.277 Mol) in 320 ml THF werden innerhalb von ca. 15 min bei 25°C 40 g (0.277 Mol) 2-Ethylhexansäure zugetropft und die Reaktionsmischung 30 min bei 50°C gerührt (Lösung 2). Lösung 1 wird bei 0°C zu Lösung 2 gegeben und anschließend wird für 1.5h auf 50°C erwärmt. Die klare Lösung wird auf 0°C abgekühlt und mit 25 g (0.833 Mol) Paraformaldehyd versetzt. Nach 10 min wird 18h bei RT nachgerührt. Es wird mit gesättigter wässriger Lösung von Ammoniumchlorid versetzt und mit 6N HCI auf pH = 1-2 eingestellt. Die organische Phase wird abgetrennt, die wässrige Phase 1x mit MTBE nachextrahiert und die vereinigten organischen Phasen am Rotationsverdampfer eingeengt. Die Ausbeute an reinem Produkt liegt bei 71 % (GC).

### Beispiel 9 (2-Ethyl-2-Hydroxymethyl-Hexansäure)

Zu einer Lösung von 46 ml Diisopropylamin (0.327 Mol) in 320 ml THF werden bei 0°C 113 ml (0.305 Mol) n-Butyl-Lithium (2.7 M in Heptan) zugetropft und 30 min bei 0°C gerührt (Lösung 1). Zu einer Suspension von 7 g NaH 95 % (0.277 Mol) in 320 ml THF werden innerhalb von ca. 15 min bei 25°C 40 g (0.277 Mol) 2-Ethylhexansäure zugetropft und die Reaktionsmischung 30 min bei 50°C gerührt (Lösung 2). Lösung 1 wird bei 0°C zu Lösung 2 gegeben und anschließend wird für 1.5h auf 50°C erwärmt. Die klare Lösung wird auf -20°C abgekühlt und mit 25 g (0.833 Mol) Paraformaldehyd versetzt. Nach 10 min wird 18h bei RT nachgerührt. Es wird mit gesättigter wässriger Lösung von Ammoniumchlorid versetzt und mit 6N HCI auf pH = 1-2 eingestellt. Die org. Phase wird abgetrennt, die wässrige Phase einmal mit MTBE nachextrahiert und die vereinigten organischen Phasen am Rotationsverdampfer eingeengt. Die Ausbeute an reinem Produkt liegt bei 70 % (GC).

## Patentansprüche

1. Verfahren zur Herstellung von α,α-Dialkyl-α-Hydroxymethyl-Carbonsäurederivaten oder deren Salzen der allgemeinen Formel (II) 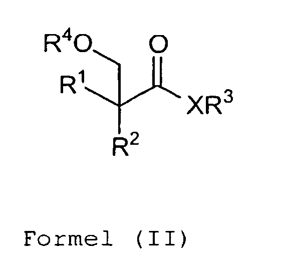 wobei
R¹ und R² unabhängig voneinander ausgewählt werden aus der Gruppe enthaltend lineare oder verzweigte Alkyl-Reste und Aralkyl-Reste,
R³ ausgewählt wird aus der Gruppe enthaltend Wasserstoff, gegebenenfalls substituierte lineare oder verzweigte Alkyl-, Aryl-, Aralkyl-Reste, Silyl-Reste, Alkalimetall, Erdalkalimetall und Ammonium,
R⁴ für Wasserstoff, eine Hydroxyl-Schutzgruppe, Alkalimetall oder Erdalkalimetall steht,
X ausgewählt wird aus der Gruppe enthaltend Sauerstoff, Schwefel und NR⁵, wobei
R⁵ wiederum ausgewählt wird aus der Gruppe enthaltend Wasserstoff, lineare oder verzweigte Alkyl-, Aryl-, Aralkyl-Reste und Silyl-Reste,
durch Umsetzung von α,α-Dialkyl-Carbonsäurederivaten oder deren Salze der allgemeinen Formel (I) 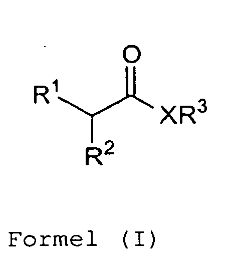 mit Formaldehyd in Gegenwart einer Amid-Base in einem oder mehreren Lösungsmitteln, **dadurch gekennzeichnet, dass** die Umsetzung in einem Temperaturbereich von -40°C bis zum Siedepunkt des verwendeten Lösungsmittels oder Lösungsmittelgemisches erfolgt.

2. Verfahren nach Anspruch 1, **dadurch gekennzeichnet, dass** α,α-Dialkyl-Carbonsäureester oder α,α-Dialkyl-Carbonsäuren oder deren Salze als Verbindungen der allgemeinen Formel (I) umgesetzt werden, wobei X für Sauerstoff (O) steht und R³ ausgewählt wird aus der Gruppe enthaltend Wasserstoff, gegebenenfalls substituierte lineare oder verzweigte Alkyl-, Aryl-, Aralkyl-Reste, Silyl-Reste, Alkalimetall, Erdalkalimetall und Ammonium.

3. Verfahren nach Anspruch 2, **dadurch gekennzeichnet, dass** X für Sauerstoff (O) steht und R³ ausgewählt wird aus der Gruppe enthaltend Wasserstoff, Lithium, Natrium, Kalium, Magnesium, Calcium und Tetraalkylammonium, Methyl, Ethyl, n-Propyl, isoPropyl, n-Butyl, iso-Butyl, tert-Butyl, Benzyl und Trimethylsilyl.

4. Verfahren nach einem oder mehreren der Ansprüche 1 bis 3, **dadurch gekennzeichnet, dass** 1 bis 3 Äquivalente der Amid-Base bezogen auf das α,α-Dialkyl-Carbonsäurederivat oder deren Salze der allgemeinen Formel (I) eingesetzt werden.

5. Verfahren nach einem oder mehreren der Ansprüche 1 bis 4, **dadurch gekennzeichnet, dass** das Lösungsmittel ausgewählt wird aus der Gruppe enthaltend Tetrahydrofuran, Diethylether, MTBE, Dibutylether, Toluol, Methylenchlorid, Pentan, Hexan, Heptan und Petrolether sowie deren Mischungen.

6. Verfahren nach einem oder mehreren der Ansprüche 1 bis 5, **dadurch gekennzeichnet, dass** als Amid-Basen Metallamide der allgemeinen Formel (III)
M(NR⁶R⁷)ₙ Formel (III)
eingesetzt werden, wobei
M ausgewählt wird aus der Gruppe enthaltend Lithium, Natrium, Kalium, Magnesium und Calcium und
R⁶ und R⁷ unabhängig voneinander ausgewählt werden aus der Gruppe enthaltend Wasserstoff, lineare oder verzweigte Alkyl-, Aryl-, Aralkyl-Reste und Silyl-Reste und
n in Abhängigkeit der Wertigkeit von M für 1 oder 2 steht.

7. Verfahren nach einem oder mehreren der Ansprüche 1 bis 6, **dadurch gekennzeichnet, dass** Natriumhexamethyldisilazid, Lithiumhexamethyldisilazid, Lithiumdiisopropylamid oder Lithiumtetramethylpiperidin als Amid-Basen eingesetzt wird.

8. Verfahren nach einem oder mehreren der Ansprüche 1 bis 7, **dadurch gekennzeichnet, dass** Formaldehyd in Form von gasförmigem Formaldehyd, Paraformaldehyd oder Trioxan eingesetzt wird.

9. Verfahren nach einem oder mehreren der Ansprüche 1 bis 8, **dadurch gekennzeichnet, dass** die Umsetzung bei einer Temperatur von -30°C bis +60°C durchgeführt wird.

## Claims

1. Process for preparing a,a-dialkyl-a-hydroxymethylcarboxylic acid derivatives or salts thereof of the general formula (II) 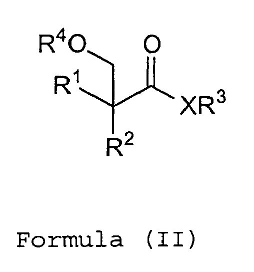 where
R¹ and R² are each independently selected from the group comprising linear or branched alkyl radicals and aralkyl radicals,
R³ is selected from the group comprising hydrogen, optionally substituted linear or branched alkyl, aryl, aralkyl radicals, silyl radicals, alkali metal, alkaline earth metal and ammonium,
R⁴ is hydrogen, a hydroxyl protecting group, alkali metal or alkaline earth metal,
X is selected from the group comprising oxygen, sulfur and NR⁵, where
R⁵ is in turn selected from the group comprising hydrogen, linear or branched alkyl, aryl, aralkyl radicals and silyl radicals,
by reacting α,α-dialkylcarboxylic acid derivatives or salts thereof of the general formula (I) 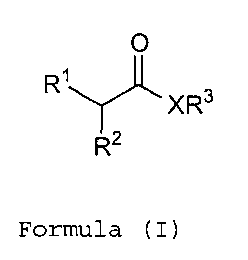 with formaldehyde in the presence of an amide base in one or more solvents, which comprises effecting the reaction in a temperature range from -40°C up to the boiling point of the solvent or solvent mixture used.

2. Process according to claim 1, **characterized in that** α,α-dialkylcarboxylic esters or α,α-dialkylcarboxylic acids or salts thereof are converted as compounds of the general formula (I), where X is oxygen (O) and R³ is selected from the group comprising hydrogen, optionally substituted linear or branched alkyl, aryl, aralkyl radicals, silyl radicals, alkali metal, alkaline earth metal and ammonium.

3. Process according to claim 2, **characterized in that** X is oxygen (O) and R³ is selected from the group comprising hydrogen, lithium, sodium, potassium, magnesium, calcium and tetraalkylammonium, methyl, ethyl, n-propyl, isopropyl, n-butyl, isobutyl, tert-butyl, benzyl and trimethylsilyl.

4. Process according to one or more of claims 1 to 3, **characterized in that** from 1 to 3 equivalents of the amide base based on the a,a-dialkylcarboxylic acid derivatives or salts thereof of the general formula (I) are used.

5. Process according to one or more of claims 1 to 4, **characterized in that** the solvent is selected from the group comprising tetrahydrofuran, diethyl ether, MTBE, dibutyl ether, toluene, methylene chloride, pentane, hexane, heptane and petroleum ether, and also mixtures thereof.

6. Process according to one or more of claims 1 to 5, **characterized in that** the amide bases used are metal amides of the general formula (III)
M(NR⁶R⁷)ₙ Formula (III)
where
M is selected from the group comprising lithium, sodium, potassium, magnesium and calcium and
R⁶ and R⁷ are each independently selected from the group comprising hydrogen, linear or branched alkyl, aryl, aralkyl radicals and silyl radicals and
n, depending on the valency of M, is 1 or 2.

7. Process according to one or more of claims 1 to 6, **characterized in that** the amide bases used are sodium hexamethyldisilazide, lithium hexamethyldisilazide, lithium diisopropylamide or lithium tetramethylpiperidine.

8. Process according to one or more of claims 1 to 7, **characterized in that** formaldehyde is used in the form of gaseous formaldehyde, paraformaldehyde or trioxane.

9. Process according to one or more of claims 1 to 8 **characterized in that** the reaction is carried out at a temperature of from -30°C to +60°C.

## Revendications

1. Procédé pour la préparation de dérivés d'acides α,α-dialkyl-α-hydroxyméthyl-carboxyliques ou leurs sels de formule générale (II) 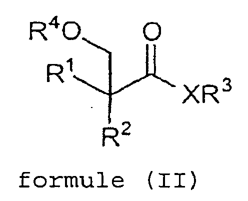 où
R¹ et R² sont choisis, indépendamment l'un de l'autre, dans le groupe contenant les radicaux alkyle et aralkyle linéaires ou ramifiés,
R³ est choisi dans le groupe contenant hydrogène, les radicaux alkyle, aryle, aralkyle le cas échéant substitués, linéaires ou ramifiés, les radicaux silyle, les métaux alcalins, les métaux alcalino-terreux et l'ammonium,
R⁴ représente hydrogène, un groupe de protection d'un groupe hydroxyle, un métal alcalin ou un métal alcalino-terreux,
X est choisi dans le groupe contenant oxygène, soufre et NR⁵, où
R⁵ est à nouveau choisi dans le groupe contenant hydrogène, les radicaux alkyle, aryle, aralkyle linéaires et ramifiés et les radicaux silyle,
par transformation de dérivés d'acides α,α-dialkylcarboxyliques ou leurs sels de formule générale (I) 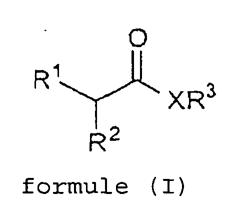 avec du formaldéhyde en présence d'une base d'amide dans un ou plusieurs solvants, **caractérisé en ce que** la transformation est réalisée dans une plage de température de -40°C jusqu'au point d'ébullition du solvant ou du mélange de solvants utilisé.

2. Procédé selon la revendication 1, **caractérisé en ce qu'**on transforme des esters d'acides α,α-dialkyl-carboxyliques ou des acides α,α-dialkyl-carboxyliques ou leurs sels comme composés de formule générale (I), où X représente oxygène (O) et R³ est choisi dans le groupe contenant hydrogène, les radicaux alkyle, aryle, aralkyle le cas échéant substitués, linéaires ou ramifiés, les radicaux silyle, les métaux alcalins, les métaux alcalino-terreux et l'ammonium.

3. Procédé selon la revendication 2, **caractérisé en ce que** X représente oxygène (O) et R³ est choisi dans le groupe contenant hydrogène, lithium, sodium, potassium, magnésium, calcium et tétraalkylammonium, méthyle, éthyle, n-propyle, iso-propyle, n-butyle, iso-butyle, tert-butyle, benzyle et triméthylsilyle.

4. Procédé selon l'une ou plusieurs des revendications 1 à 3, **caractérisé en ce qu'**on utilise 1 à 3 équivalents de la base d'amide par rapport au dérivé d'acide α,α-dialkyl-carboxylique ou ses sels de formule générale (I).

5. Procédé selon l'une ou plusieurs des revendications 1 à 4, **caractérisé en ce que** le solvant est choisi dans le groupe contenant le tétrahydrofuranne, le diéthyléther, le MTBE, le dibutyléther, le toluène, le chlorure de méthylène, le pentane, l'hexane, l'heptane et l'éther de pétrole ainsi que leurs mélanges.

6. Procédé selon l'une ou plusieurs des revendications 1 à 5, **caractérisé en ce qu'**on utilise comme bases d'amide des amides métalliques de formule générale (III)
M(NR⁶R⁷)ₙ formule (III)
où
M est choisi dans le groupe contenant le lithium, le sodium, le potassium, le magnésium et le calcium et
R⁶ et R⁷ sont choisis, indépendamment l'un de l'autre, dans le groupe contenant hydrogène, les radicaux alkyle, aryle, aralkyle linéaires ou ramifiés et les radicaux silyle et
n vaut, en fonction de la valence de M, 1 ou 2.

7. Procédé selon l'une ou plusieurs des revendications 1 à 6, **caractérisé en ce qu'**on utilise de l'hexaméthyldisilazide de sodium, de l'hexaméthyldisilazide de lithium, du lithiumdiisopropylamide ou de la lithiumtétraméthylpipéridine comme bases d'amide.

8. Procédé selon l'une ou plusieurs des revendications 1 à 7, **caractérisé en ce que** le formaldéhyde est utilisé sous forme de formaldéhyde gazeux, de paraformaldéhyde ou de trioxane.

9. Procédé selon l'une ou plusieurs des revendications 1 à 8, **caractérisé en ce que** la transformation est réalisée à une température de -30°C à +60°C.
